# EUROPEAN PATENT APPLICATION

(11) **EP 4 269 460 A1**
(43) Date of publication of application: **01.11.2023**
(21) Application number: 21910495.7
(22) Date of filing: 14.12.2021
(51) Int. Cl.: C08F 290/14, A61Q 1/12, A61Q 17/04, C08L 83/14, C08L 101/00, C08L 101/16, A61K 8/891, A61K 8/895

(54) **NOVEL SILICONE ELASTOMER PARTICLES, AND COSMETIC COMPOSITION AND OTHER APPLICATIONS**

(30) Priority: 25.12.2020 JP 2020216520
(71) Applicant: Dow Toray Co., Ltd., Tokyo 140-8617 (JP)
(72) Inventor: WAKITA, Mari, Ichihara-shi Chiba 299-0108 (JP); TANIGUCHI, Hiroko, Ichihara-shi Chiba 299-0108 (JP); KANZAKI, Yasue, Ichihara-shi Chiba 299-0108 (JP); SUGIURA, Tsunehito, Ichihara-shi Chiba 299-0108 (JP)
(74) Representative: Murgitroyd & Company
(86) International application number: PCT/JP2021/046142
(87) International publication number: WO 2022/138346

(57) **Abstract**

[Problem]

To provide novel silicone elastomer particles that have higher oil absorbency than conventional products and are less prone to aggregation, making the particles superior in handling workability as a cosmetic raw material and imparting a superior tactile sensation and feel during use to a cosmetic product.

[Resolution Means]

Silicone elastomer particles, having a structure in which at least two silicon atoms in a silicone elastomer particle are crosslinked by a divalent organic group having a partial structure formed by radical polymerization of vinyl acetate, as expressed by -{CH₂-CH(COOCH₃)}ₜ- (where t is a number of 1 or more), as well as a use thereof. In particular, the novel silicone elastomer particles have a crosslinked structure that is active with respect to biodegradability. Therefore, primary particles thereof are expected to have a property of disintegrating with the generation of non-crosslinked siloxane molecules due to a decomposition reaction of microorganisms and the like in the natural world.

## Description

### TECHNICAL FIELD

The present invention relates to novel silicone elastomer particles that have higher oil absorbency than conventional products and are less prone to aggregation, making the particles easy to handle as a cosmetic raw material and imparting a superior tactile sensation and feel during use to a cosmetic product. Furthermore, the novel silicone elastomer particles have a crosslinked structure that is active with respect to biodegradability. Therefore, primary particles thereof are expected to have a property of disintegrating with the generation of non-crosslinked siloxane molecules due to a decomposition reaction of microorganisms and the like in the natural world, and thus are expected to behave as biodegradable silicone elastomer particles. Furthermore, the present invention relates to a cosmetic raw material, cosmetic material composition, organic resin additive, and other applications containing the silicone elastomer particles, as well as to a method of manufacturing the silicone elastomer particles.

### BACKGROUND ART

Silicone elastomer particles are cured from an addition reaction-curable or condensation reaction-curable silicone composition, and the particle diameter and oil absorbency thereof vary depending on the manufacturing method. In general, there is a limit to refinement in crushing a cured product into particulates. Therefore, a preferred manufacturing method is to obtain silicone particles with a smaller particle diameter by curing and reacting particulate material of a crosslinkable silicone composition. However, even if the primary particles are fine, they tend to aggregate into secondary particles over time, and the aggregated particles are not easily re-dispersed into primary particles. This is due to a phenomenon that once primary particles bond with each other as aggregated particles, the bond is difficult to separate (dissociate).

When highly cohesive silicone particles are added to a solvent or the like, the silicone particles do not disperse at the primary particle size and become secondary aggregated particles or aggregates thereof, resulting in insufficient dispersion and the inability to prepare a uniform mixture. As a result, compositions containing silicone particles cannot fully demonstrate the characteristics of silicone particles, and in particular, have insufficient handling workability, storage stability, and blending stability in the system as cosmetic raw materials or organic resin additives. Note that in general, flexible rubberlike particles with low hardness, such as silicone elastomer particles, tend to aggregate over time.

When these silicone elastomer particles are used as cosmetic raw materials, it has been widely proposed that the tactile sensation and feel during use of cosmetic materials can be improved by adding a liquid oil agent (cosmetic material oil agent) after the silicone elastomer particles, or by combining a liquid oil agent and the silicone elastomer particles (for example, Patent Document 1). However, as described above, silicone elastomer particles tend to aggregate over time, and this property affects the oil absorbency of the silicone particles, which may result in insufficient improvement in the expected tactile sensation and feel during use of the cosmetic material.

Meanwhile, as silicone particles having superior dispersibility, high lipophilicity, and superior storage stability, the present applicant has proposed silicone particles containing an alkylene group having 4 to 20 carbon atoms, which is obtained by curing a crosslinkable composition for forming silicone particles having a low amount of silicon atom-bonded hydrogen atoms per unit mass and containing an alkenyl group having 4 to 20 carbon atoms, such as a hexenyl group or the like, as described in Patent Document 2. However, although these silicone particles have improved dispersion stability and the like as a cosmetic raw material, there is still room for improvement in terms of the tactile sensation and feel during use when combined with a liquid oil agent in cosmetic materials as described above.

In addition thereto, the applicant has focused on essential issues in conventional silicone elastomer particles. In other words, conventional silicone elastomer particles are formed through a crosslinking reaction of organopolysiloxane raw materials by hydrosilylation reactions or the like. The crosslinked structure is chemically stable, and even if these silicone elastomer particles were to be released into nature, it is undeniable that they would remain in nature without decomposing, at least for a short period of time, just like so-called microplastics. Therefore, there seems to be a latent desire in the market for silicone elastomer particles that perform well enough to smoothly replace or substitute existing silicone elastomer particles and that are expected to be highly biodegradable, in order to reduce risk to the global environment.

On the other hand, while polymer powders or composite particles using vinyl acetate and the like as raw materials are disclosed in Patent Documents 3 and 4, these documents do not describe nor suggest any reactions involving a polyorganosiloxane and silicone elastomer particles involving a crosslinking reaction using vinyl acetate and the like (in particular, Patent Document 3 is use for core-shell or hollow structures). Moreover, there is no description or suggestion that the use of these raw materials imparts beneficial properties to silicone elastomer particles, and there is no commonality of technical problems and resolution means with the present invention.

### RELATED ART DOCUMENTS

### Patent Documents

Patent Document 1: Japanese Unexamined Patent Application H07-316014
Patent Document 2: International Patent Publication WO2017/191798
Patent Document 3: Japanese Unexamined Patent Application 2012-007059
Patent Document 4: Japanese Unexamined Patent Application 2002-241427

### SUMMARY OF THE INVENTION

### PROBLEM TO BE SOLVED BY THE INVENTION

In order to solve the aforementioned problem, an object of the present invention is to provide: silicone elastomer particles that, when added to a cosmetic material composition or the like, have high oil absorbency, are flexible, and have an effect of improving tactile sensation and feel during use in cosmetic materials and the like, and that, compared with conventional silicone elastomer particles, have reduced aggregation over time and smaller average secondary particle diameters, which results in superior dispersibility and handling workability as a cosmetic raw material and the like, storage stability, and blending stability in systems; and a manufacturing method thereof. Furthermore, an object of the present invention is to provide a cosmetic raw material, an organic resin additive, and other applications with excellent handling workability and the like by using the silicone elastomer particles. Furthermore, an object of the present invention is to provide a cosmetic material composition containing the silicone elastomer particles, with excellent feel during use, storage stability, and the like.

Furthermore, an object of the present invention is to provide: silicone elastomer particles that, in addition to performance equal to or better than conventional silicone elastomer particles, can be expected to be biodegradable, thereby reducing potential risks to the global environment, allowing for industrially sustainable and stable use, and making it possible to promote the material as biodegradable and eco-friendly to users and consumers in general who are concerned about global environmental impact.

### MEANS FOR SOLVING THE PROBLEM

As a result of extensive studies to solve the aforementioned problem, the present inventors discovered that the problem can be solved using silicone elastomer particles having a structure in which at least two silicon atoms in a silicone elastomer particle are crosslinked by a divalent organic group having a partial structure formed by radical polymerization of vinyl acetate, as expressed by -{CH₂-CH(COOCH₃)}ₜ- (where t is a number of 1 or more), thereby achieving the present invention. Such silicone elastomer particles can be easily obtained by crosslink reacting in water crosslinking reactive silicone emulsified particles obtained by emulsifying a crosslinking reactive silicone composition by a radical polymerization reaction in water, the crosslinking reactive silicone composition at least containing: (a) an organopolysiloxane having at least two organic groups containing a (meth)acryloxy group of at least one type selected from organic groups containing a methacryloxy group and organic groups containing an acryloxy group in a molecule; (b) vinyl acetate; and (c) a radical initiator, and being suitable as means for solving the problem of the present invention.

Furthermore, the present inventors discovered that the silicone elastomer particles described above can be used in cosmetic raw materials and other applications, and that the problem described above can be solved by a cosmetic material composition and the like containing the same, thereby achieving the present invention.

### EFFECT OF THE INVENTION

Compared to conventionally known silicone elastomer particles, the silicone elastomer particles according to the present invention have higher oil absorbency with respect to the same oil agent, while at the same time they can achieve a hard, smooth surface condition, which greatly suppresses aggregation as secondary particles. Therefore, when added to a cosmetic material composition or the like, the particles have high oil absorbency, are flexible, and have a more excellent effect on improving the tactile sensation and feel during use of the cosmetic material and the like, and the small aggregated particle diameter provides high dispersibility in a system and inhibits aggregation over time. Thus, the particles have excellent handling workability as a cosmetic raw material and the like, storage stability, and blending stability in a system. Furthermore, the silicone elastomer particles according to the present invention can be used to provide a cosmetic raw material, an organic resin additive, and other applications containing the silicone elastomer particles. Furthermore, a cosmetic material composition containing the silicone elastomer particles according to the present invention can provide a cosmetic material with excellent feel during use, storage stability, and the like.

Furthermore, the silicone elastomer particles according to the present invention are designed such that the divalent organic group having a partial structure formed by radical polymerization of vinyl acetate is active with respect to biodegradable reactions, a crosslinked structure formed between silicon atoms in the silicone elastomer particle is at least partially cleaved in a biodegradable environment, and primary particles of the silicone elastomer particles have a property of disintegrating with the generation of a polyorganosiloxane with a non-crosslinked structure. Therefore, the silicone elastomer particles according to the present invention are expected to be biodegradable, which reduces the risk to the global environment, and appeal to users and consumers in general, who are concerned about global environment impact, as an eco-friendly material that can be used with a considerable sense of ease.

### BRIEF DESCRIPTION OF THE DRAWINGS

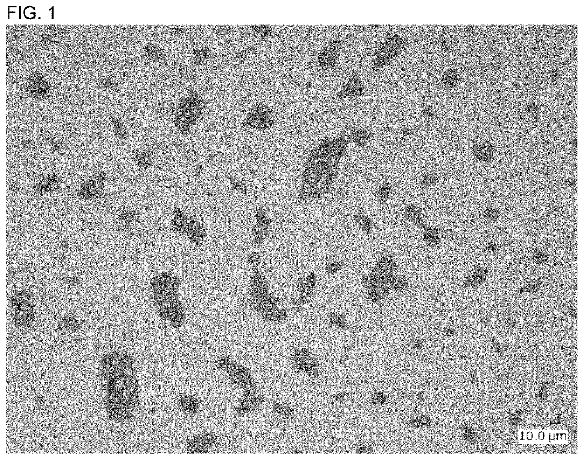

FIG. 1 is an image of silicone elastomer particles (Example 1) according to the present invention observed with a digital microscope (device name: KEYENCE model number VH-6000).

### PREFERRED MODE FOR CARRYING OUT THE INVENTION

The silicone elastomer particles of the present invention, applications thereof including cosmetic raw materials in particular, a manufacturing method thereof, as well as a cosmetic material composition and an organic resin (including paint and coating agent) containing the same will be described in detail below.

The silicone elastomer particles of the present invention are characterized in having a structure in which at least two silicon atoms in a silicone elastomer particle are crosslinked by providing a partial structure formed by radical polymerization of vinyl acetate, as expressed by:

-{CH₂-CH(COOCH₃)}ₜ-

In the formula, t is a number of 1 or more, and the average value of t described later may be a number within a range of 0.5 to 20 and more preferably a number within a range of 1 to 15.

Herein, the actual silicone elastomer particles have a chemically amphotropic distribution, depending on the mass ratio of the amount of vinyl acetate as a raw material and the amount of other radical polymerizable functional groups in the composition, particularly organic groups containing a (meth)acryloxy group, which will be discussed later. For example, if the crosslinked structure described above is formed by a radical polymerization reaction between vinyl acetate and the organic group containing a (meth)acryloxy group bonded to a silicon atom, the amount of vinyl acetate is expected to be one or more on average with respect to the amount of the organic group containing a (meth)acryloxy group, but the vinyl acetate and (meth)acryloxy group can each independently radically polymerize; therefore, a structure selected from:
· Partial structures resulting from radical polymerization between vinyl acetate and vinyl acetate,
· Partial structures resulting from radical polymerization between vinyl acetate and a (meth)acryloxy group, and
· Partial structures resulting from radical polymerization between (meth)acryloxy groups
may be mixed in the silicone elastomer particles.

Herein, the expression chemically amphotropic includes partial structures formed by radical polymerization of vinyl acetate, expressed by -{CH₂-CH(COOCH₃)}ₜ-, within any silicon-silicon crosslinked structure in the silicone elastomer particles, by a reaction involving at least radical polymerization between vinyl acetate and the (meth)acryloxy group. On the other hand, in consideration of the silicone elastomer particles as a whole, the value of t in the partial structure containing a vinyl acetate polymer in the crosslinked structure between silicon atoms is expressed as an average value. For example, the average value of t in the partial structure at the crosslinking site is determined by the mass ratio of the amount of vinyl acetate and the amount of organic groups containing a (meth)acryloxy group bonded to a silicon atom in the composition.

As described later, in the present invention, the mass ratio of the amount of vinyl acetate and organic groups containing a (meth)acryloxy group bonded to a silicon atom in the composition is within a range of 0.5 to 20, preferably 1 to 15, more preferably 3 to 12, and particularly preferably 5 to 10. Therefore, the average value of t is also preferably a number corresponding thereto.

The silicone elastomer particles of the present invention preferably further contain a polyorganosiloxane structure expressed by:

-(R¹₂SiO)ₙ-

(where R¹ represents an unsubstituted alkyl group having 1 to 20 carbon atoms, an alkyl group having 1 to 20 carbon atoms substituted with a halogen atom, an aryl group having 6 to 22 carbon atoms, or a hydroxyl group, and n is a number ranging from 1 to 1000).

This is a linear polysiloxane structure derived from component (a), described later, which provides the silicone elastomer particles with an appropriate degree of hardness and flexibility.

Industrially, R¹ preferably independently represents a methyl group or phenyl group, and n is more preferably a number ranging from 50 to 800, and more preferably 75 to 750.

The silicone elastomer particles according to the present invention are preferably obtained by curing crosslinking reactive silicone emulsified particles by a crosslinking reaction. Particularly preferably, the silicone elastomer particles of the present invention are defined by the manufacturing process thereof, and are obtained by crosslinking reacting in water crosslinking reactive silicone emulsified particles obtained by emulsifying a crosslinking reactive silicone composition by a radical polymerization reaction in water, the crosslinking reactive silicone composition at least containing: (a) an organopolysiloxane having at least three organic groups containing a (meth)acryloxy group of at least one type selected from organic groups containing a methacryloxy group and organic groups containing an acryloxy group in a molecule; (b) vinyl acetate; and (c) a radical initiator.

Furthermore, silicone elastomer particles obtained via such a manufacturing process may further improve the appearance, spreadability, and tactile sensation of the cosmetic material, particularly when used as a cosmetic raw material, and particles obtained via this manufacturing method tend to be more suitable for solving the problem of the present invention. Thus, one suitable form for achieving the technical effect of the present invention can be and is suitably defined by the manufacturing process.

The silicone elastomer particles according to the present invention are not particularly limited in terms of the average primary particle diameter thereof, but from the perspective of imparting a smooth tactile sensation and feel during use to the cosmetic material, not causing appearance defects and the like, storage stability and blending stability as a cosmetic raw material, and the like, the average particle diameter measured by a laser diffraction scattering method is preferably within a range of 0.5 to 20 µm, more preferably within a range of 0.5 to 15 µm. Note that the particle diameter of the silicone elastomer particles can be controlled according to the crosslinking reactive silicone emulsified particles and crushing/classification process of the resulting silicone elastomer particles.

The shape of the silicone elastomer particles according to the present invention includes, for example, spherical, regular spherical shape, elliptical, and irregular shapes, and in particular, spherical and regular spherical shapes are preferred. Spherical silicone elastomer particles can be easily obtained by the method described later, in which the particles are prepared in the form of an aqueous suspension and dried using a vacuum dryer, hot air circulation oven, or spray dryer.

Furthermore, in the present invention, the crosslinking reactive silicone composition used to form the silicone elastomer particles is preferably within a range of 10 to 80 when cured in a sheet form, as measured by a JIS A hardness tester as defined in JIS K6301. If the JIS-A hardness of the rubber sheet measured by curing the crosslinking reactive silicone composition into a sheet form is within the range above, the resulting silicone elastomer particles will have sufficiently low aggregation and will tend to have exceptional flowability, dispersibility, smoothness, silkiness and a soft tactile sensation. Furthermore, by selecting the JIS-A hardness described above, it is possible to design or predict to some extent the feel during use, tactile sensation, and handling workability when added to a cosmetic material, and to improve stress relaxation properties when added to an organic resin. Note that when the silicone elastomer particles according to the present invention are used as a cosmetic raw material or stress relief agent or the like for organic resins, silicone elastomer particles having a JIS-A hardness in a range of 30 to 80, and particularly 50 to 80, described above are particularly preferably used.

Optionally, the silicone elastomer particles of the present invention may further contain a structure in which some or all of the surface thereof is covered with one or more types selected from organopolysiloxane resins, silica, and other silicone elastomer particles. Such coating may be expected to further reduce aggregation, control oil absorbency, improve tactile sensation, and the like.

Optionally, the silicone elastomer particles of the present invention may be mesoporous structures with micropores.

Optionally, the silicone elastomer particles of the present invention may contain an oil agent that is liquid at 40°C. The oil agent can be easily included in the silicone elastomer particles by emulsifying together in the crosslinking reactive silicone composition described later, and the inclusion of the oil agent may be expected to further reduce aggregation, control oil absorbency, improve tactile sensation, and the like.

Optionally, the silicone elastomer particles of the present invention may further have a structure in which at least two silicon atoms configuring the particles are crosslinked by a silalkylene group having 2 to 20 carbon atoms formed by a hydrosilylation reaction involving an alkenyl group having 2 to 20 carbon atoms. These structures can be easily achieved by using an alkenyl group-containing organopolysiloxane, organohydrogen polysiloxane, and hydrosilylation reactive catalyst in combination. Furthermore, for example, by using an alkenyl group having 4 or more carbon atoms, such as a hexenyl group or the like, further reduction of aggregation, control of oil absorbency, improvement of tactile sensation may be expected due to the combination of the silalkylene structure. However, if biodegradability is a primary objective, the silicone elastomer particles of the present invention are preferably substantially free of structures containing a silalkylene group.

### [Crosslinking reactive silicone composition for Use in Formation of Silicone Elastomer Particles]

More specifically, the silicone elastomer particles of the present invention can be obtained by crosslinking (curing) a crosslinking reactive silicone composition containing the following components by a radical polymerization reaction.
(a) An organopolysiloxane having at least three organic groups containing a (meth)acryloxy group of at least one type selected from organic groups containing a methacryloxy group and organic groups containing an acryloxy group in a molecule,
(b) Vinyl acetate, and
(c) A radical initiator

Component (a) is an organopolysiloxane having at least three organic groups containing a (meth)acryloxy group in a molecule, the structure of which is not particularly limited and may be one or more type of structure selected from linear, cyclic, reticulate, or partially branched linear structures. However, a linear organopolysiloxane is particularly preferred. The viscosity of component (a) is preferably a viscosity that allows the crosslinkable composition above to be dispersed in water. Specifically, it is preferably in a range of 20 to 100,000 mPa·s at 25°C, and particularly preferably in a range of 20 to 10,000 mPa s.

From the perspective of tactile sensation, dispersibility and handling workability of the silicone elastomer particles, component (a) is preferably a linear organopolysiloxane in which the amount of the dimethylsiloxane units expressed by the formula: (CH₃)₂SiO is 90 mol% or more of all siloxane units other than the siloxane units at molecular terminals. Similarly, from the perspective of improving the oil absorbency of the resulting silicone elastomer particles and the like, a cyclic or chain organopolysiloxane with a low degree of polymerization (degree of polymerization of 3 to 20) may be removed from component (a) beforehand by stripping or the like.

Furthermore, when the component (a) is a linear organopolysiloxane, the silicone elastomer particles according to the present invention, when placed in a biodegradable environment, have an advantage that when the silicone elastomer particles are broken down by cleavage of the crosslinked structure, they are more likely to break down into non-crosslinkable, linear organopolysiloxanes, thus reducing the environmental impact and environmental risk.

A functional group of at least one type selected from organic groups containing a methacryloxy group and organic groups containing an acryloxy group is included in the organic group containing a (meth)acryloxy group in component (a). Furthermore, in the present specification, when the term "(meth)acryloxy group" is used, it shall refer to "a functional group selected from methacryloxy groups and acryloxy groups". Component (a) must have at least three or more organic groups containing a (meth)acryloxy group on average in a molecule to form a crosslinked structure in a radical reaction with component (b). If only two or fewer organic groups containing a (meth)acryloxy group are present in a molecule on average, a sufficient crosslinked structure may not be formed, and practical silicone elastomer particles may not be obtained.

More specifically, the organic group containing a (meth)acryloxy group is a (meth)acryloxy group bonded to a silicon atom via a divalent organic group, and one or more functional group expressed by the following is exemplified.

-R²-O-C(=O)-C(R³)=CH₂

{where R² represents an alkylene group having 1 to 20 carbon atoms or a divalent linking group expressed by

(CH₂)ₚ-Si(CH₃)₂-O-Si(CH₃)₂-(CH₂)_{q}

(p and q in the formula are each a number ranging from 1 to 20), and
R³ represents a hydrogen atom or a methyl group.}

The alkylene group, which is R² in the formula, may industrially be an alkylene group having 2 to 10 carbon atoms, and examples include propylene groups, butylene groups, hexylene groups, and the like. Furthermore, a divalent linking group expressed by (CH₂)ₚ-Si(CH₃)₂-O-Si(CH₃)₂-(CH₂)_{q} is a divalent linking group having a siloxane converter structure, and industrial examples include linking groups where p and q are each independently a number from 3 to 6.

Suitably, component (a) is preferably a linear organopolysiloxane expressed by the following structural formula.

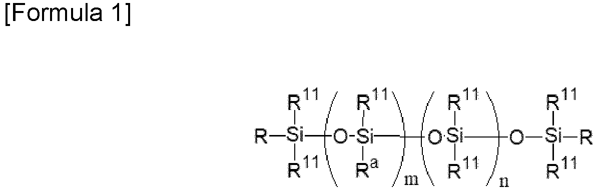

In Formula (1), each R¹¹ is independently an unsubstituted or halogen atom-substituted alkyl group (for example, a methyl group, or the like) having 1 to 20 carbon atoms, an aryl group (for example, a phenyl group, or the like) having 6 to 22 carbon atoms, or a hydroxyl group, and a methyl group or a phenyl group is preferable from an industrial point of view. R^{a} represents an organic group containing a (meth)acryloxy group, and is particularly preferably a (meth)acryloxy group bonded to a silicon atom by the aforementioned alkylene group or divalent linking group with a siloxane converter structure. R independently is a group represented by R¹¹ or Ra. m is a number of 1 or more, and n is a number of 1 or more. However, component (a) contains at least three organic groups containing a (meth)acryloxy group represented by R^{a} in a molecule, and therefore, when m = 1, R must both be R^{a}. In other words, the linear organopolysiloxane expressed by the structural formula above has a organic group containing a (meth)acryloxy group represented by R^{a} at one end site and a side chain site, a side chain site only, or both end sites and a side chain site of a siloxane molecule thereof, and preferably contain at least three organic groups containing a (meth)acryloxy group in a molecule.

m+n is the degree of siloxane polymerization of linear organopolysiloxane molecules excluding end siloxane structures. From the perspective of handling workability as a raw material and the ability to break down into fine linear siloxane molecules during emulsification biodegradation, m + n is preferably within a range of 10 to 800, more preferably 20 to 600, and particularly preferably 30 to 500. Furthermore, the viscosity of component (a) is particularly preferably a number between 20 and 10,000 mPa·s at 25°C.

The vinyl acetate (b) is a crosslinking agent of component (a), which provides the characteristic crosslinked structure of the silicone elastomer particles according to the present invention, and is itself a radical polymerizable monomer component that forms a polymer or copolymer structure by radical polymerization. By causing a reaction between the organic group containing a (meth)acryloxy group in component (a) and the vinyl acetate to form a radical copolymer, a divalent organic group having a partial structure expressed by

-{CH₂-CH(COOCH₃)}ₜ-

(where t is a number of 1 or more)
is formed between organopolysiloxane main chains derived from component (a).

The crosslinked structure between polysiloxane chains containing the partial structure is provided within the silicone elastomer particle. Therefore, compared to conventionally known silicone elastomer particles, the silicone elastomer particles according to the present invention have higher oil absorbency with respect to the same oil agent, while at the same time they can achieve a hard, smooth surface condition, which greatly reduces aggregation as secondary particles.

Furthermore, the crosslinked portion is active with respect to biodegradable reactions, a crosslinked structure formed between silicon atoms in the silicone elastomer particle is at least partially cleaved in a biodegradable environment, and primary particles of the silicone elastomer particles have a property of disintegrating with the generation of a polyorganosiloxane with a non-crosslinked structure. Herein, if component (a) is a linear polyorganosiloxane as described above, the biodegradable reaction facilitates the breakdown of the silicone elastomer particles into linear polyorganosiloxane molecules, which are broken down into fine liquid components, not solid powders with minute particle diameter sizes, as in macroplastics. Therefore, it is expected to have little impact or burden on the global environment, as it is unlikely to cause problems of bioaccumulation through the food chain or accumulation/deposition in the environment.

Herein, the amount of component (b) used is preferably within a range of 0.5 to 30, more preferably 1 to 20, even more preferably 3 to 15, and particularly preferably 5 to 10, in terms of the mass ratio of the amount of component (b) to the amount of organic groups containing a (meth)acryloxy group in component (a). If the amount of component (b) used is within the range above, a crosslinked structure derived from a radical polymer of vinyl acetate with an appropriate average length between polyorganosiloxane structures is obtained, which enables particles to achieve a smooth surface state with moderate hardness and low tack, and effectively inhibits aggregation between particles. On the other hand, if the amount of component (b) used is less than the lower limit above, crosslinking may be insufficient. If the amount of component (b) used exceeds the upper limit above, emulsion breakdown and the like are likely to occur during a curing reaction, and thus silicone elastomer particles may not be obtained.

Component (c) is a radical initiator and is a component for promoting a radical polymerization reaction or radical copolymerization reaction of components (a) and (b) described above. A conventionally known compound commonly used in radical polymerization methods is used as the radical initiator, and specific examples include 2,2'-azobis(isobutyronitrile), 2,2'-azobis(2-methylbutyronitrile), 2,2'-azobis(2,4-dimethylvaleronitrile), and other azo-based compounds; benzoyl peroxide, lauroyl peroxide, tert-butylperoxy benzoate, tert-butylperoxy-2-ethylhexanoate, tert-hexylperoxy-2-ethylhexanoate, and other organic peroxides; and potassium persulfate, sodium persulfate, ammonium persulfate, and other persulfates. One type of this radical initiator may be used alone, or two or more types may be mixed and used together.

The amount of component (c), which is a radical initiator, used is preferably within a range of 0.1 to 5 parts by mass relative to 100 parts by mass of the total of components (a) and (b) above. In particular, when component (c) is a water-soluble persulfate such as potassium persulfate or the like, component (c) has an advantage of being extremely easy to add and react when crosslink reacting in water crosslinking reactive silicone emulsified particles, which are obtained by emulsifying a crosslinking reactive silicone composition in water via a radical polymerization reaction.

The timing of adding component (c) to the crosslinkable composition can be selected according to the method of forming the silicone elastomer particles, and may be in the form of adding to the composition in advance, or in the form of supplying component (a) or component (b) from a different spray line, then adding component (c) to one of the components, and then mixing during spraying. The silicone elastomer particles in the present invention are preferably formed via an aqueous suspension, which is formed via emulsification into water, and component (c) may be added in advance to the crosslinking reactive silicone composition, or an emulsion containing component (c) may be added separately to the water.

A chain transfer agent can be optionally added during the polymerization reaction of the crosslinking reactive silicone composition above. Specific examples of the chain transfer agent include mercapto compounds such as 2-mercaptoethanol, butyl mercaptan, n-dodecyl mercaptan, 3-mercaptopropyl trimethoxysilane, polydimethylsiloxanes having a mercaptopropyl group, and the like; and halides such as methylene chloride, chloroform, carbon tetrachloride, butyl bromide, 3-chloropropyl trimethoxysilane, and the like.

The crosslinking reactive silicone composition above may contain one or more polymerization inhibitors from the perspective of preventing unintended side reactions and the like. For example, one or more types selected from hindered phenol-based polymerization inhibitors, hydroquinonebased polymerization inhibitors, and catechol-based polymerization inhibitors may be included. The amount used can be selected as appropriate, but the total concentration of the polymerization inhibitor is preferably 50 ppm by mass or less, and more preferably 30 ppm by mass or less, with respect to the sum of components (a) to (c) above.

The crosslinking reactive silicone composition may include a component other than the components above to the extent that the technical effects of the present invention are not impaired. For example, the composition may include: n-hexane, cyclohexane, n-heptane, or other aliphatic hydrocarbons; toluene, xylene, mesitylene, or other aromatic hydrocarbons; tetrahydrofuran, dipropyl ether, or other ethers; an organic solvent such as acetone, methyl ethyl ketone, methyl isobutyl ketone, other ketones, or the like; a phenolic antioxidant, quinone antioxidant, amine antioxidant, phosphorus antioxidant, phosphite antioxidant, sulfuris antioxidant, thioether antioxidant, or other antioxidant; a triazole light stabilizer, benzophenone light stabilizer, or other light stabilizer; a phosphate ester flame retardant, halogen flame retardant, phosphorus flame retardant, antimony flame retardant, or other flame retardant; one or more antistatic agents including cationic surfactants, anionic surfactants, non-ionic surfactants, and the like; a dye; a pigment; or the like.

The silicone elastomer particles of the present invention may optionally further have: (A) a structure in which part or all of a surface thereof is coated by one or more substance selected from organopolysiloxane resins, silica, and other silicone elastomer particles; (B) a mesoporous structure; (C) a structure containing an oil agent that is liquid at 40°C; and (D) a structure crosslinked by a silalkylene group having 2 to 20 carbon atoms, and a combination of arbitrary components providing these structures may be used.

### [Hardness of Silicone Elastomer]

Although the hardness of the silicone elastomer particles cannot be measured directly, the hardness can be measured indirectly by curing the crosslinkable silicone composition used to form the silicone elastomer particles serving as a raw material thereof. Specifically, the crosslinking reactive silicone composition is cured in a sheet form without emulsifying in water, and the hardness of the silicone elastomer sheet can be measured by a JIS A hardness tester as defined in JIS K6301. The hardness of the silicone elastomer according to the present invention varies depending on the type of the crosslinkable silicone composition, the amount of components (a)/(b) used, and the crosslink density, but is preferably within a range of 10 to 80. In addition, the preferred hardness is as described above.

### [Formation of Silicone Elastomer Particles and Manufacturing Method Thereof]

An example of a method of obtaining the silicone elastomer particles according to the present invention includes a method including a step of curing crosslinking reactive silicone emulsified particles, which are obtained by emulsifying in water with the crosslinking silicone composition used to form the silicone elastomer particles above, in the presence of the radical initiator (c) to obtain spherical silicone elastomer particles.

More specifically, the silicone elastomer particles according to the present invention can and preferably are prepared using a manufacturing method including the following steps (I) and (II).
Step (I):
   a step of emulsifying
      (a) an organopolysiloxane having at least two organic groups containing a (meth)acryloxy group,
      (b) vinyl acetate, and
      (c) a radical initiator
   in water to form crosslinking reactive silicone emulsified particles; and
Step (II):
   a step of curing the crosslinking reactive silicone emulsified particle obtained in step (I) in the presence of the radical initiator (c) to obtain silicone elastomer particles.

The crosslinkable silicone composition used to form the silicone elastomer particles can be uniformly mixed using a mixer or other mechanical force.

In this method, silicone elastomer particles can be obtained by emulsifying and curing the aforementioned crosslinkable silicone composition in a surfactant aqueous solution. In addition, the particle diameter can be easily adjusted by adjusting the emulsion particle diameter. Examples of these surfactants include nonionic, anionic, cationic, and betaine types. The particle diameter of the resulting silicone elastomer particles varies depending on the type and content of the surfactant. In order to prepare silicone elastomer particles having a small particle diameter, the amount of the surfactant added is preferably within a range of 0.5 to 50 parts by mass with respect to 100 parts by mass of the crosslinkable silicone composition.

An emulsifier is preferably used to uniformly disperse the crosslinking silicone composition in water in the form of crosslinking reactive silicone emulsified particles. Examples of the emulsifying machine include a homomixer, a paddle mixer, a Henschel mixer, a homo-disper, a colloid mill, a propeller agitator, a homogenizer, an in-line continuous emulsifier, an ultrasonic emulsifier, and a vacuum kneading machine.

The aqueous dispersion of the crosslinking reactive silicone emulsified particles prepared by the method above can then be heated or left at room temperature to cure the crosslinking reactive silicone emulsified particles in the aqueous dispersion to prepare a aqueous dispersion of silicone elastomer particles. When such a aqueous dispersion is heated, the heating temperature is preferably 100°C or lower from the perspective of radical polymerization reactivity, and is particularly preferably 10 to 95°C. Furthermore, examples of a method of heating the aqueous dispersion containing the crosslinking reactive silicone emulsified particles include a method of directly heating the water-based dispersion, and a method of adding the aqueous dispersion to hot water. The crosslinking reaction causes the liquid crosslinking reactive silicone emulsified particles to cure in water, forming a aqueous dispersion of silicone elastomer particles.

The resulting silicone elastomer particles of the present invention can be used as-is as an aqueous dispersion (aqueous suspension). In particular, the aqueous suspension form may be and is preferably used in cosmetic raw materials and the like. When added to a cosmetic material that uses an aqueous solution as a dispersion medium (such as hair cosmetic materials and the like), the silicone elastomer particles may be easily and uniformly dispersed to achieve a desired performance and feel during use by being added as an aqueous dispersion containing the silicone elastomer particles of the present invention.

Suitably, the silicone elastomer particles according to the present invention can be isolated by removing water from the aqueous dispersion of silicone elastomer particles. The method of removing water from the aqueous dispersion includes, for example, drying using a vacuum dryer, a hot air circulation oven, or a spray dryer. Note that the heating and drying temperature of the spray dryer must be set appropriately based on the heat resistance, crosslinking temperature, and the like of the silicone elastomer particles. Note that in order to prevent secondary aggregation of the resulting microparticles, the temperature of the silicone elastomer particles is preferably controlled to be equal to or lower than the glass transition temperature thereof. The silicone elastomer particles thus obtained can be recovered by a cyclone, a bag filter, or the like. Note that as a pretreatment for this operation, the dispersion may be concentrated by a method of heating and dehydration, filtration separation, centrifugation, decantation, or the like, and if necessary, the dispersion may be washed with water.

The silicone elastomer particles of the present invention may be surface treated, if necessary, to further improve the aggregation suppression effect of the silicone elastomer particles of the present invention. Furthermore, surface treatment with another known hydrophilic or hydrophobic treatment agent or the like may be applied. Optionally, the resulting silicone elastomer particles may be further coated with silica or other inorganic microparticles, silicone resin, or the like, in whole or in part on the surface thereof, as described above. Furthermore, the resulting silicone elastomer particles may be crushed or pulverized by a mechanical force if necessary, or classified using a known technique.

### [Cosmetic Raw Material and Cosmetic Material Composition]

The silicone elastomer particles of the present invention are useful as a cosmetic raw material. When added to a cosmetic material composition or the like, the silicone elastomer particles have high oil absorbency, are flexible, and have a more excellent effect on improving the tactile sensation and feel during use of the cosmetic material and the like. Furthermore, the small secondary particle diameter thereof makes it highly dispersible in a system compared to conventional silicone elastomer particles. Moreover, aggregation is inhibited over time. Thus, it is remarkably excellent in handling workability as a cosmetic raw material and the like, storage stability, and blending stability in a system.

In particular, the silicone elastomer particles of the present invention have smaller secondary particle diameters than known silicone particles and are less likely to aggregate over time, making them not only easier to disperse in a system but also superior in handling workability and storage stability, as no prior agitation or crushing operation is required when adding, even after long-term storage. In addition, it has excellent uniform dispersibility in cosmetic materials, and can be expected to mix more uniformly with pigments and the like included in cosmetic materials to further improve the appearance and feel during use of cosmetic materials. In addition, the silicone elastomer particles of the present invention have both relatively high oil absorbency, which is an oil absorption property not found in conventional silicone elastomer particles, and uniform dispersibility in other cosmetic raw materials (particularly, oil-based raw materials), including lipophilic materials. Therefore, the degree of freedom in formulation design is high, and the silicone elastomer particles do not absorb oil-based raw materials over time when added to a cosmetic material, causing thickening or changes in texture. Furthermore, when applied to the skin or hair, they have the advantage of suppressing the oiliness and stickiness of the cosmetic material, providing a smooth spread and soft tactile sensation, and offering an excellent feel during use.

Furthermore, the silicone elastomer particles of the present invention are active with respect to biodegradable reactions while providing performance that is equal or superior to conventionally known silicone elastomer particles. Moreover, a crosslinked structure formed between silicon atoms in the silicone elastomer particle is at least partially cleaved in a biodegradable environment, and primary particles of the silicone elastomer particles have a property of disintegrating with the generation of a polyorganosiloxane with a non-crosslinked structure. Therefore, the silicone elastomer particles are a low risk and low impact material for the global environment. Furthermore, use is possible as a replacement to conventionally known silicone elastomer particles, making it extremely versatile.

The cosmetic material composition containing the silicone elastomer particles of the present invention are not particularly limited in type, and examples include products such as soaps, body shampoos, facial cleansing creams, and other cleaning cosmetic materials; toners, creams/milky lotions, packs, and other base cosmetic products; powders, foundation, and other base makeup cosmetic materials; lipstick, cheek rouge, eye shadow, eyeliners, mascara, and other facial cosmetic materials; nail polish and other makeup cosmetic materials; shampoos, hair rinses, hairdressing material, hair growth promoters, hair dye, and other hair cosmetic materials; perfume and eau de cologne, and other aromatic cosmetic materials; toothpastes; bath agents; and hair removal agents, shaving lotions, antiperspirants/deodorants, sunscreen agents, and other special cosmetic materials. Examples of the dosage forms of these cosmetic material compositions include aqueous liquid, oil-based liquid, emulsion, cream, foam, semi-solid, solid, and powder. These cosmetic material compositions can also be used by spraying.

In these cosmetic material compositions, the amount of the silicone elastomer particles described above is preferably within a range of 0.5 to 99.0 mass% in the cosmetic material composition, and particularly preferably within a range of 1.0 to 95 mass%. This is because if the amount of the silicone elastomer particles described above exceeds the upper limit of the range above, the effect as a cosmetic material is lost, and if the amount is below the lower limit of the range above, feel during use and the like of the cosmetic material composition and the like is less likely to be improved.

With respect to cosmetic material compositions (in particular, each formulation example) containing silicone particles (silicone rubber powder and the like) or silicone composite particles, which have been proposed in Patent Document 1 (Japanese Unexamined Patent Application H07-316014), Patent Document 2 (International Patent Publication WO2017/191798), and Patent Document 3 (Japanese Unexamined Patent Application H02-243612) as well as Japanese Unexamined Patent Application 2011-105663, Japanese Unexamined Patent Application 2011-168634, Japanese Unexamined Patent Application 2011-102354, and Japanese Unexamined Patent Application 2014-122316 described above, the silicone elastomer particles of the present invention can be used in place of a part or all of these silicone-based particles, and there are cases where the feel during use as well as the production efficiency of the cosmetic material compositions proposed in these Patent Documents can be further improved. Note that it goes without saying that examples of cosmetic material compositions containing silicone particles (silicone rubber powder or the like) or silicone composite particles that can be blended with the silicone elastomer particles of the present invention are not limited to the above, and formulations may be designed in which some or all of silicone particle components in commercially available cosmetic materials are replaced by the silicone elastomer particles of the present invention, using a technique common to a person of ordinary skill in the art.

Furthermore, the silicone elastomer particles of the present invention can be applied to replace some or all of these silicone-based particles with respect to the applications and formulations of cosmetic material compositions disclosed in the aforementioned Patent Documents and the like, and the use therein is encompassed within the scope of the invention of the present application. As an example, the silicone elastomer particles of the present invention may be and are preferably used in combinations of arbitrary components, such as cosmetic product media (aqueous media or oil-based media), oil-based media (including oil agents and volatile oil agents), water, colorants, pigments, ultraviolet light blocking components, alcohols, water-soluble polymers, film-forming agents, oil agents, oil-soluble gelling agents, organically modified clay minerals, surfactants, resins, salts, moisturizers, preservatives, antimicrobial agents, antioxidants, pH adjusters, chelating agents, refreshing agents, anti-inflammatory agents, skin brightening agents (such as whitening agents, cell activators, skin roughness improvement agents, blood circulation promoters, skin astringents, and anti-seborrheic agents), vitamins, amino acids, nucleic acids, hormones, inclusion compounds, bioactive substances, medicament active components, perfumes, and the like, by selecting a method or quantitative range similar to those disclosed in Patent Document 2 (International Patent Publication WO2017/191798).

In particular, the silicone elastomer particles of the present invention have superior handling workability, storage stability, dispersibility, and high oil absorption properties that are equal or superior to conventionally known silicone particles, silicone composite particles coated with silsesquioxane, and silicone particles containing an oil agent. Therefore, a particularly preferred appearance, feel during use, and the like can be achieved in the following:
(1) Cosmetic material compositions and formulations containing oil-based media such as oil agents and the like (oil-based cosmetic raw materials);
(2) Cosmetic material compositions and formulations containing lipophilic ultraviolet light blocking components (such as octyl paramethoxycinnamate and the like); and
(3) Cosmetic material compositions and formulations containing inorganic powders such as colorants, pigments, or the like.

These specific formulations are further described in detail in the Examples and thereafter.

In addition thereto, the silicone elastomer particles of the present invention can be easily designed for aqueous dispersions. Therefore, in aqueous cosmetic material compositions and formulations, the silicone elastomer particles provide excellent formulation design freedom and blending stability, and thus can achieve a suitable feel during use. These specific formulations are further described in detail in the Examples and thereafter.

The cosmetic material of the present invention can be easily manufactured by simply uniformly mixing the cosmetic product raw material of the present invention and other cosmetic product raw materials as described above. As mixing means, various mixing and kneading devices normally used in the manufacture of cosmetic products can be used. Examples of such devices include a homomixer, a paddle mixer, a Henschel mixer, a homodisper, a colloidal mixer, a propeller agitator, a homogenizer, an in-line continuous emulsifier, an ultrasonic emulsifier, and a vacuum kneading machine.

### [Organic Resin Additives and Organic Resins, Paints, and Coating Agents]

The silicone elastomer particles of the present invention are also extremely useful as an organic resin additive due to the properties described above. Specifically, the silicone elastomer particles of the present invention have superior uniform dispersibility in organic resins and, if desired, excellent stress relief properties, and the like. In addition, the particles have very excellent handling workability and storage stability because aggregation is less likely to occur even after long-term storage. Furthermore, a member, paint film, or coating film obtained by curing an organic resin containing the silicone elastomer particles has improved flexibility (including the softness of a coating layer), durability, and adhesion to and followability of the substrate, is particularly pliable, and has superior thermal shock resistance. Therefore, it is extremely useful as a highly functional organic resin, paint, or coating agent for use in electronic materials.

### [Organic Resin]

A curable organic resin composition or a thermoplastic resin is suitably exemplified as an organic resin containing the silicone elastomer particles of the present invention. Of these, curable resins are suitable for electronic materials such as semiconductor substrates and the like. More specifically, examples of the curable organic resin composition include, phenolic resin, formaldehyde resin, xylene resin, xylene-formaldehyde resin, ketoneformaldehyde resin, furan resin, urea resin, imide resin, melamine resin, alkyd resin, unsaturated polyester resin, aniline resin, sulfone-amide resin, silicone resin, epoxy resin, and copolymer resins of these resins, and two or more of these curable resins can be combined. In particular, the curing resin is preferably at least one type selected from the group consisting of an epoxy resin, a phenolic resin, an imide resin, and a silicone resin. Examples of the curing mechanisms of such curable resins are thermal curing, high energy beam curing such as ultraviolet light, radiation, and the like, moisture curing, condensation reaction curing, and addition reaction curing. The properties of such curable resins at 25°C are not limited, and may be in either a liquid state or a solid state that softens upon heating.

Another optional component such as a curing agent, curing promoter, filler, photosensitizer, higher fatty acid metal salt, ester wax, plasticizer, or the like can be added to the organic resin containing the silicone elastomer particles of the present invention. Examples of curing agents include: organic acids such as carboxylic acids, sulfonic acids, and the like and anhydrides thereof; organic hydroxy compounds; organic silicon compounds having a silanol group, an alkoxy group, or a halogeno group; and primary or secondary amino compounds, and two or more types can be combined. Examples of the curing promoter include: tertiary amine compounds, organic metal compounds such as aluminum, zirconium, and the like; organophosphorus compounds such as phosphine and the like; other heterocyclic amine compounds, boron complex compounds, organic ammonium salts, organic sulfonium salts, organic peroxides, and catalysts for hydrosilylation. Examples of these fillers include: fibrous fillers such as glass fiber, asbestos, alumina fiber, ceramic fiber composed of alumina and silica, boron fiber, zirconia fiber, silicon carbide fiber, metal fiber, polyester fiber, aramid fiber, nylon fiber, phenolic fiber, natural animal and plant fiber, and the like; powdered fillers such as fused silica, precipitated silica, fumed silica, calcined silica, zinc oxide, calcined clay, carbon black, glass beads, alumina, talc, calcium carbonate, clay, aluminum hydroxide, barium sulfate, titanium dioxide, aluminum nitride, silicon carbide, magnesium oxide, beryllium oxide, Kaolin, mica, zirconia, and the like. Two or more of these can be combined. In the case of epoxy resins, it is particularly preferable to include an amine curing agent.

The silicone elastomer particles of the present invention may be added as an additive to a thermoplastic resin other than those described above, and may be used as a modifier of physical properties such as surface lubricants, stress relief agents, and the like, or modifiers of optical properties such as light scattering agents and the like. The type of thermoplastic resin is not particularly limited, and may be at least one polymer selected from a group consisting of: polycarbonate resins; polyester resins; polyether resins; polylactic acid resins; polyethylene, polypropylene, ethylene-propylene copolymers, and other polyolefin resins; polystyrene resins; styrene copolymers; tetrafluoroethylene and other fluorine-based polymers; polyvinyl ethers; and cellulose-based polymers, or a composite resin containing a combination of these. The silicone resin coated silicone elastomer particles of the present invention can be uniformly dispersed in these thermoplastic resins (including master batches) using a mixing device such as a biaxial or single-axis extruder, a kneader mixer, or the like, and may be molded into a desired shape, such as a film form or the like, for use.

The added amount of the silicone elastomer particles of the present invention may be selected as appropriate according to the physical properties required of the organic resin, but is generally within a range of 0.1 to 30 parts by mass with respect to 100 parts by mass of the organic resin, and may be within a range of 0.5 to 10 parts by mass. The reason is that if the amount of the particles added is less than the lower limit, the performance such as stress relief properties for the resin or the like may become insufficient, and the pliability and thermal shock resistance of the resulting cured organic resin product may decrease, and in particular, the thermal shock resistance tends to decrease after moisture absorption. On the other hand, if the amount exceeds the upper limit, the organic resin or the paint/coating agent after blending may become thickened and the handling workability may decrease, and the mechanical properties of the resulting organic resin cured product tend to decrease.

Furthermore, the silicone elastomer particles of the present invention are superior in stress relief when added to an organic resin, and thus may be added to an epoxy resin or the like for printed wiring boards to form a prepreg. Furthermore, a copper foil containing filler particles for a printed wiring board provided with a resin layer containing the silicone elastomer particles of the present invention on one side of the copper foil may be formed and used for a copper clad laminate (CCL) application.

### [Paints, Coating Agents]

Examples of paints and coating agents containing the silicone elastomer particles of the present invention include ambient temperature curing types, ambient temperature drying types, and heat curing types, with examples based on the properties thereof including aqueous types, oil-based types, and powdered types, and examples based on the vehicle resin including polyurethane resin paint, butyral resin paint, long oil phthalate resin paint, alkyd resin paint, amino alkyd resin paint made up of amino resin and alkyd resin, epoxy resin paint, acrylic resin paint, phenol resin paint, silicone modified epoxy resin paint, silicone modified polyester resin paint, and silicone resin paint.

The added amount of the silicone elastomer particles of the present invention can be selected as appropriate according to the physical properties required for the paint/coating agent, but in order to impart uniform and soft matting properties to the resulting coating film, the added amount is preferably within a range of 0.1 to 150 parts by mass with respect to 100 mass parts of solid content of the paint, more preferably within a range of 0.1 to 100 parts by mass, even more preferably 0.1 to 50 parts by mass, and particularly preferably 0.1 to 20 parts by mass. If the amount of the particles added is less than the lower limit, performance such as matting, adhesion, stress relief properties, and the like of the coating film may be insufficient. If the amount of the particles exceeds the upper limit, the organic resin and the paint/coating agent after blending may become thickened and the handling workability may decrease.

The paints and coating agents containing the silicone elastomer particles of the present invention may contain: alcohols such as methanol, ethanol, and the like; ketones such as methyl ethyl ketone, methyl isobutyl ketone, and the like; esters such as ethyl acetate, butyl acetate, cellosolve acetate, and the like; amides such as N,N-dimethylformamide and the like; olefins such as hexane, heptane, octane, and the like; organic solvents such as toluene, xylene, and other aromatic hydrocarbons; known inorganic fillers such as reinforcing silica and the like; organic fillers; curing promoters; silane coupling agents; carbon black and other pigments; dyes; antioxidants; thickeners containing a high molecular weight compound; flame retardants; and weather resistance imparting agents.

### [As Eco-Friendly Material]

As described above, unlike conventional non-biodegradable thermoplastic resin particles and silicone particle materials, the silicone elastomer particles of the present invention are expected to be biodegradable in a biodegradable environment, where crosslinked structures formed between silicon atoms within the silicone elastomer particles are at least partially cleaved, and primary particles of the silicone elastomer particles have a property of disintegrating with the generation of a polyorganosiloxane with a non-crosslinked structure. Therefore, use is possible as an "eco-friendly" cosmetic raw material with low environmental burden and environmental risk that complies with regulations on microplastics and the like, and is expected to appeal as a biodegradable and "eco-friendly" material to users and consumers who are concerned about global environmental impact.

### [EXAMPLES]

The silicone elastomer particles of the present invention and the manufacturing method thereof will be described in detail based on examples and comparative examples. However, the present invention is not limited only to these examples. Viscosity in the examples is the value at 25°C. The properties of each silicone particle were measured as follows. Note that tn the examples and the like, unless otherwise specified, silicone particles is a generic term for particles made of a silicone cured product (cured silicone particles), and does not include emulsions.

### [Average Primary Particle Diameter of Emulsion Particles]

The emulsion before the addition of the radical polymerization initiator (= potassium persulfate) was measured using a laser diffraction particle diameter distribution analyzer (LS-230 from Beckman Coulter), and the median diameter (particle diameter corresponding to 50% of the cumulative distribution, 50% particle diameter) was used as the average particle diameter.

### [Average Secondary Particle Diameter of Silicone Particles (Powder)]

Using ethanol as a dispersion medium, the particle diameter of the cured silicone particles was measured with a laser diffraction particle diameter distribution analyzer (Mastersizer 3000 from Malvern Panalytical), and the median diameter of the cured silicone particles in ethanol (particle diameter corresponding to 50% of the cumulative distribution, D90, µm) and the arithmetic dispersion (particle diameter distribution SD, µm2) values were obtained. For the measurement sample, cured silicone particles (1 g) and ethanol (100 mL) were dispersed in a 300 mL cup using stirring blades and an ultrasonic vibrator.

### [Measurement Method of Powder Oil Absorption]

1 g of cured silicone particles was placed in a 100 mL beaker, and while slowly stirring the silicone particles with a glass rod, squalane was added drop by drop until the cured silicone particles and oil formed a uniform paste. The ratio of drops of oil to cured silicone particles was used as the oil absorption amount (weight%). The oil absorption amount was determined similarly for decamethylcyclopentasiloxane and mineral oil (HICALL K-230 available from Kaneda Co., Ltd.).

The average formula of component (A) used in the examples and comparative examples are listed below.
In the following formulas, Vi represents a vinyl group expressed by CH₂=CH-, Me represents a methyl group expressed by CH₃-, X represents a methacrylic group expressed by CH₃-C(CH₂)-COO-, and Y represents a methacrylicmodified group expressed by CH₂=C(CH₃)-COO-C₃H₆-(Me₂SiO)-(SiMe₂)-C₆H₁₂-.

[Formula 1-1] Me₃SiO-(Me₂SiO)_{262.2}-(MeSiO(C₃H₆-X))_{4.6}-SiMe₃

[Formula 1-2] Me₃SiO-(Me₂SiO)₁₄₆-(MeSiO(C₃H₆-X))_{3.4}-SiMe₃

[Formula 1-3] Y-Me₂SiO-(Me₂SiO)_{132.5}-(Me(Y)SiO)_{2.5}-SiMe₂-Y

### [Example 1]

A polyorganosiloxane expressed by the average formula of [Formula 1-1] and a vinyl acetate monomer (manufactured by FUJIFILM Wako Pure Chemical Corporation) were uniformly mixed at a mass ratio of 90:10 at room temperature. The composition was then dispersed in an aqueous solution at 25°C containing 0.4 parts by mass of polyoxyethylene alkyl (C12-14) ether and 40 parts by mass of pure water, further emulsified uniformly by a colloid mill, and then diluted by adding 514 parts by mass of pure water and 2.0 parts by mass of polyoxyethylene alkyl (C12-14) ether to prepare an emulsion. After heating in a 1 L flask to 60°C, an aqueous solution of 0.5 g potassium persulfate (manufactured by Sigma-Aldrich) dissolved in 9.5 g water was added dropwise over one minute. The emulsion was stirred at 60°C for three hours at 100 rpm to perform radical polymerization to prepare a uniform aqueous suspension of silicone rubber particles. The aqueous suspension was then filtered and the residue was dried in an oven at 70°C for three hours to obtain silicone elastomer particles. The average secondary particle diameter of the resulting silicone elastomer particles was 30.6 µm.

### [Example 2]

Silicone elastomer particles were obtained in the same manner as in Example 1, except that polyorganosiloxane expressed by the average formula of [Formula 1-2] was used instead of the polyorganosiloxane expressed by the average formula of [Formula 1-1]. The average secondary particle diameter of the resulting silicone elastomer particles was 18.0 µm.

### [Example 3]

A polyorganosiloxane expressed by the average formula of [Formula 1-3] and a vinyl acetate monomer (manufactured by FUJIFILM Wako Pure Chemical Corporation) were uniformly mixed at a mass ratio of 90:10 at room temperature. The composition was then dispersed in an aqueous solution at 25°C containing 0.4 parts by mass of polyoxyethylene alkyl (C12-14) ether and 50 parts by mass of pure water, further emulsified uniformly by a colloid mill, and then diluted by adding 504 parts by mass of pure water and 2.0 parts by mass of polyoxyethylene alkyl (C12-14) ether to prepare an emulsion. The silicone elastomer particles were obtained in the same manner as in Example 1. The average secondary particle diameter of the resulting silicone elastomer particles was 17.8 µm.

### [Example 4]

A polyorganosiloxane expressed by the average formula of [Formula 1-1] and a vinyl acetate monomer (manufactured by FUJIFILM Wako Pure Chemical Corporation) were uniformly mixed at a mass ratio of 80:20 at room temperature. Otherwise, the silicone elastomer particles were obtained in the same manner as in Example 1. The average secondary particle diameter of the resulting silicone elastomer particles was 37.5 µm.

### [Example 5]

A polyorganosiloxane expressed by the average formula of [Formula 1-1] and a vinyl acetate monomer (manufactured by FUJIFILM Wako Pure Chemical Corporation) were uniformly mixed at a mass ratio of 70:30 at room temperature. Otherwise, the silicone elastomer particles were obtained in the same manner as in Example 1. The average secondary particle diameter of the resulting silicone elastomer particles was 41.0 µm.

### [Comparative Example 1]

Silicone elastomer particles were obtained in the same manner as in Example 1, except that the vinyl acetate monomer was not used, and 100 parts by mass of the polyorganosiloxane expressed by the average formula of [Formula 1-1] was used. The average secondary particle diameter of the resulting silicone elastomer particles was 50.0 µm.

### [Comparative Example 2]

Silicone elastomer particles were obtained in the same manner as in Example 1, except that the vinyl acetate monomer was not used, and 100 parts by weight of the polyorganosiloxane expressed by the average formula of [Formula 1-2] was used. The average secondary particle diameter of the resulting silicone elastomer particles was 24.1 µm.

Furthermore, the silicone elastomer particles obtained in Example 1 were observed with a digital microscope (device name: Keyence model number VH-6000) under the following conditions, and an image thereof is depicted in FIG. 1.

The properties of Examples 1 to 4 and Comparative Examples 1 to 2 are shown in Table 1.

**[Table 1]**

| | Average primary particle diameter (µm) | Average secondary particle diameter (µm) | Oil absorption amount (Squalane) g/g | Oil absorption amount (Cyclopentasiloxane) g/g | Oil absorption amount (Mineral oil) g/g |
|---|---|---|---|---|---|
| Example 1 | 5.6 | 30.6 | 0.42 | 1.51 | 0.42 |
| Example 2 | 7.2 | 18.0 | 0.34 | 1.16 | 0.39 |
| Example 3 | 5.9 | 17.8 | 0.51 | 1.88 | 0.55 |
| Example 4 | 4.8 | 37.5 | 0.44 | 1.57 | 0.50 |
| Example 5 | 3.0 | 41.0 | 0.44 | 1.64 | 0.52 |
| Comparative Example 1 | 7.0 | 50.0 | 0.35 | 1.61 | 0.45 |
| Comparative Example 2 | 5.5 | 24.1 | 0.32 | 1.22 | 0.36 |

The silicone elastomer particles according to Examples 1 to 5 above have a relatively high amount of oil absorption, particularly for squalane and mineral oil, but have a small average secondary particle diameter in contrast to the average primary particle diameter thereof. Therefore, superior dispersion stability is expected as compared to the silicone elastomer particles of Comparative Examples 1 and 2. Furthermore, a hard feel and smooth tactile sensation were provided. Furthermore, as depicted in FIG. 1, digital microscope observation of the silicone elastomer particles obtained in Example 1 confirmed that the particles were essentially free of aggregation and dispersibility was excellent.

### [Cosmetic Material Formulation Example]

The following are formulation examples of cosmetic materials of the present invention that can contain silicone elastomer particles, which is one aspect of the present invention. However, the present invention is not limited thereto.

### [Example 6 and Comparative Examples 3/4]

Panelists compared and evaluated the feel during use of loose powder in which the silicone elastomer particles in the compositions listed in Table 3 were used.

### Evaluation of Tactile Sensation

The slipperiness of the samples applied to inner forearms of 18 panelists was evaluated using the criteria in Table 2 below.

**[Table 2]**

| Evaluation results | Evaluation Indicators |
|---|---|
| ○ | 12 or more of 18 respondents said the slipperiness was favorable |
| Δ | 7 to 11 of 18 respondents said the slipperiness was favorable |
| × | 6 or fewer of 18 respondents said the slipperiness was favorable |

**[Table 3]**

| Phase | Component | Product name and supplier | Comparative Example 3 | Example 6 | Comparative Example 4 |
|---|---|---|---|---|---|
| A | Silicone elastomer particles of Example 1 | | | 10 | |
| | Silicone elastomer particles of Comparative Example 1 | | | | 10 |
| | Bis(hydroxyethylpropyl) dimethicone | DOWSIL^{™} 5562 Carbinol Fluid | 6 | 6 | 6 |
| B | Talc | JA-46R available from ASADA MILLING CO., LTD. | 76 | 76 | 76 |
| | Silylated silica | DOWSIL^{™} VM-2270 Aerogel Fine Particle | 2 | 2 | 2 |
| | Kaolin | | 1.5 | 1.5 | 1.5 |
| | Titanium oxide | SI Titanium CR-50 available from Miyoshi Kasei, Inc. | 3.92 | 3.92 | 3.92 |
| | Iron oxide yellow | SI-YELLOW-LLXLO available from Miyoshi Kasei, Inc. | 0.46 | 0.46 | 0.46 |
| | Iron oxide red | SA-Bengara Cloisonne available from Miyoshi Kasei, Inc. | 0.09 | 0.09 | 009 |
| | Iron oxide black | SA-Black BL-100 available from Miyoshi Kasei, Inc. | 0.02 | 0.02 | 0.02 |
| Evaluation of tactile sensation | | | △ | ○ | × |

### (Method of preparation)

1. Mix phase A.
2. Mix phase B.
3. Phases A and B are stirred until uniform.

As shown in Table 2, loose powder using the silicone elastomer particles of the present invention (Example 1) was evaluated to have favorable slipperiness, unlike Comparative Example 3, which did not contain elastomer particles, as well as loose powder using other particles (Comparative Example 1).

### [Example 6 and Comparative Examples 3/4]

The panelists compared and evaluated the feel during use of water-in-oil sunscreen agents in which the silicone elastomer particles in the compositions listed in Table 5 were used.

### Evaluation of Tactile Sensation

The 18 panelists evaluated the spreadability and white residue when the samples were applied to inner forearms using the criteria in Table 4 below.

**[Table 4]**

| Evaluation results | Evaluation Indicators |
|---|---|
| ○ | 12 or more of 18 respondents said spreadability is favorable and white residue is less likely to remain |
| Δ | 7 to 11 out of 18 respondents said spreadability is favorable and white residue is less likely to remain |
| × | 6 or less of 18 respondents said spreadability is favorable and white residue is less likely to remain |

**[Table 5]**

| Phase | Component | Product name and supplier | Comparative Example 5 | Example 7 | Comparative Example 6 |
|---|---|---|---|---|---|
| A | Zinc oxide dispersion | DLZ-01 available from Tayca Co., Ltd. | 30 | 30 | 30 |
| | Titanium oxide dispersion | DLT-01 available from Tayca Co., Ltd. | 20 | 20 | 20 |
| | Glyceryl tri(capric acid/caprylic acid) | Crodamol GTCC available from Croda | 6 | 6 | 6 |
| | Dimethicone | XIAMETER^{™} PMX-200 2cs | 13.5 | 13.5 | 13.5 |
| | Dimethicone | DOWSIL^{™} SH200 C Fluid 6cs | 4 | 4 | 4 |
| | Silicone emulsifiers | DOWSIL^{™} ES-5300 Formulation Aid | 2 | 2 | 2 |
| | Silicone elastomer particles of Example 1 | | | 3 | |
| | Silicone elastomer particles of Comparative Example 1 | | | | 3 |
| B | Sodium citrate | | 0.2 | 0.2 | 0.2 |
| | Sodium chloride | | 0.5 | 0.5 | 0.5 |
| | BG | | 3 | 3 | 3 |
| | Water | | 17.8 | 17.8 | 17.8 |
| C | Preservative | Euxyl PE9010 available from Schülke & Mayr | 0.7 | 0.7 | 0.7 |
| Evaluation of tactile sensation | | | × | ○ | Δ |

### (Method of preparation)

Mix phase A.
Phase B is mixed.
Phase B is slowly added while stirring Phase A.
Phase C is added to 4 above and then stirred until uniform.

As shown in Table 5, the oil-in-water sunscreen agent using the silicone elastomer particles of the present invention (Example 1) was evaluated to have favorable spreadability and less white residue, unlike Comparative Example 5, which did not contain elastomer particles, as well as loose powder using other particles (Comparative Example 1).

## Claims

1. Silicone elastomer particles, comprising a structure in which at least two silicon atoms in a silicone elastomer particle are crosslinked by a divalent organic group having a partial structure formed by radical polymerization of vinyl acetate, as expressed by:
-{CH₂-CH(COOCH₃)}ₜ-
(where t is a number of 1 or more).

2. The silicone elastomer particles according to claim 1, further comprising a polyorganosiloxane structure expressed by:
-(R¹₂SiO)n-
(where R¹ represents an unsubstituted alkyl group having 1 to 20 carbon atoms, an alkyl group having 1 to 20 carbon atoms substituted with a halogen atom, an aryl group having 6 to 22 carbon atoms, or a hydroxyl group, and n is a number ranging from 1 to 1000).

3. The silicone elastomer particles according to claim 1 or 2, obtained by crosslinking reacting in water crosslinking reactive silicone emulsified particles obtained by emulsifying a crosslinking reactive silicone composition by a radical polymerization reaction in water, the crosslinking reactive silicone composition at least comprising:
(a) an organopolysiloxane having at least three organic groups containing a (meth)acryloxy group of at least one type selected from organic groups containing a methacryloxy group and organic groups containing an acryloxy group in a molecule;
(b) vinyl acetate; and
(c) a radical initiator.

4. The silicone elastomer particles according to claim 3, wherein the organic group containing a (meth)acryloxy group in component (a) above is a functional group bonded to a silicon atom, as expressed by:
-R²-O-C(=O)-C(R³)=CH₂
{where R2 represents an alkylene group having 1 to 20 carbon atoms or a divalent linking group expressed by:
(CH₂)ₚ-Si(CH₃)₂-O-Si(CH₃)₂-(CH₂)_{q}
(p and q in the formula are each a number ranging from 1 to 20), and R³ represents a hydrogen atom or a methyl group.}.

5. The silicone elastomer particles according to claim 3 or 4, wherein the mass ratio of the amount of component (b) is within a range of 0.5 to 30 relative to the amount of the organic group containing a (meth)acryloxy group in component (a) above.

6. The silicone elastomer particles according to any one of claims 3 to 5, wherein component (a) above is a linear organopolysiloxane having in a molecule at least two organic groups containing a (meth)acryloxy group expressed by the following structural formula: 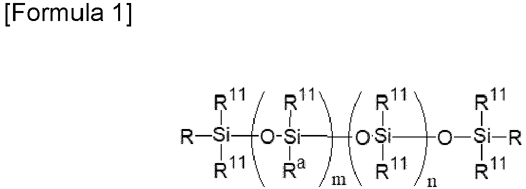 (where R¹¹ independently represents an unsubstituted alkyl group, an alkyl group having 1 to 20 carbon atoms substituted with a halogen atom, an aryl group having 6 to 22 carbon atoms, or a hydroxyl group, and R^{a} represents an organic group containing a (meth)acryloxy group. R is a group represented by R¹¹ or R^{a}, but when m = 1, both Rs are R^{a}. m is a number of 1 or more, n is a number of 1 or more, and m + n is a number ranging from 10 to 800.).

7. The silicone elastomer particles according to any one of claims 1 to 6, wherein the divalent organic group, which has a partial structure formed by radical polymerization of vinyl acetate, is formed by a radical polymerization reaction between vinyl acetate and an organic group containing a (meth)acryloxy group bonded to a silicon atom.

8. The silicone elastomer particles according to any one of claims 1 to 7, wherein the average particle diameter as measured by a laser diffraction scattering method is 0.5 to 20 µm.

9. The silicone elastomer particles according to any one of claims 1 to 8, wherein the crosslinking reactive silicone composition used in forming the silicone elastomer particles is cured in a sheet form, and the JIS-A hardness measured is within a range of 10 to 80.

10. The silicone elastomer particles according to any one of claims 1 to 8, further comprising a structure in which some or all of the surface thereof is covered with one or more types selected from organopolysiloxane resins, silica, and other silicone elastomer particles.

11. The silicone elastomer particles according to any one of claims 1 to 8, further comprising a mesoporous structure.

12. The silicone elastomer particles according to any one of claims 1 to 8, further comprising an oil agent that is liquid at 40°C.

13. The silicone elastomer particles according to any one of claims 1 to 8, further comprising a structure in which at least two silicon atoms are crosslinked by a silalkylene group having 2 to 20 carbon atoms formed by a hydrosilylation reaction involving an alkenyl group having 2 to 20 carbon atoms.

14. The silicone elastomer particles according to any one of claims 1 to 13, wherein biodegradability is provided.

15. The silicone elastomer particles according to any one of claims 1 to 13, wherein the divalent organic group having a partial structure formed by radical polymerization of vinyl acetate is active with respect to biodegradable reactions, a crosslinked structure formed between silicon atoms in the silicone elastomer particle is at least partially cleaved in a biodegradable environment, and primary particles of the silicone elastomer particles have a property of disintegrating with the generation of a polyorganosiloxane with a non-crosslinked structure.

16. A cosmetic raw material, comprising the silicone elastomer particles according to any one of claims 1 to 15.

17. A cosmetic material composition, comprising the silicone elastomer particles according to any one of claims 1 to 15.

18. An organic resin additive, comprising the silicone elastomer particles according to any one of claims 1 to 15.

19. An organic resin, comprising the silicone elastomer particles according to any one of claims 1 to 15.

20. A method of manufacturing the silicone elastomer particles according to any one of claims 3 to 15, comprising the following step (I) and step (II):
Step (I):
a step of emulsifying
(a) an organopolysiloxane having at least two organic groups containing a (meth)acryloxy group,
(b) vinyl acetate, and
(c) a radical initiator
in water to form crosslinking reactive silicone emulsified particles; and
Step (II): a step of curing the crosslinking reactive silicone emulsified particle obtained in step (I) in the presence of the radical initiator (c) to obtain silicone elastomer particles.
